# FASCICULE DE BREVET EUROPEEN

(11) **EP 3 237 893 B1**
(45) Date de publication et mention de la délivrance du brevet: **10.10.2018**
(21) Numéro de dépôt: 15823708.1
(22) Date de dépôt: 22.12.2015
(51) Int. Cl.: G01N 23/046

(54) **FANTOME DESTINE A ETRE UTILISE POUR LE CONTROLE DE LA QUALITE D'IMAGES TOMOGRAPHIQUES.**
RÖNTGENPHANTOM ZUR VERWENDUNG IN DER QUALITÄTSKONTROLLE VON TOMOGRAPHIEBILDERN
PHANTOM INTENDED FOR USE IN QUALITY CONTROL OF TOMOGRAPHIC IMAGES

(30) Priorité: 23.12.2014 FR 1463208
(43) Date de publication de la demande: 01.11.2017
(73) Titulaire: SAFRAN AIRCRAFT ENGINES, 75015 Paris (FR); Safran Landing Systems, 78140 Vélizy-Villacoublay (FR)
(72) Inventeur: GAY, Lionel, Christian, Jean-Loïc, 91330 Yerres (FR); ARSLAN, Philippe, 94140 Alfortville (FR); RAMBOURG, Yves, 91190 Gif-sur-Yvette (FR); CHANDELLE, André, 77550 Moissy-Cramayel Cedex (FR)
(74) Mandataire: Desormiere, Pierre-Louis
(86) Numéro de dépôt international: PCT/FR2015/053733
(87) Numéro de publication internationale: WO 2016/102896

(56) Documents cités:
- EP-A1- 0 875 751
- HANSON K M ET AL: "Computed tomography using proton energy loss", PHYSICS IN MEDICINE AND BIOLOGY, INSTITUTE OF PHYSICS PUBLISHING, BRISTOL GB, vol. 26, no. 6, novembre 1981 (1981-11), pages 965-983, XP020022418, ISSN: 0031-9155, DOI: 10.1088/0031-9155/26/6/001

## Description

Fantôme destiné à être utilisé pour le contrôle de la qualité d'images tomographiques.

### Arrière-plan de l'invention

La présente invention se rapporte au domaine général du contrôle de la qualité en imagerie tomographique 2D ou 3D.

Elle trouve préférentiellement mais non exclusivement une application au contrôle de la qualité d'images tomographiques de pièces réalisées en matériau composite et utilisées notamment dans le domaine aéronautique.

La tomographie est une technique couramment utilisée dans le domaine du contrôle non destructif de pièces pour obtenir une reconstruction 2D ou 3D des défauts internes d'une pièce. Cette technique permet, à l'aide d'un appareil d'imagerie, de visualiser et quantifier avec précision les caractéristiques des défauts de la pièce (position dans l'espace, taille, facteur de forme, etc.).

Par ailleurs, il est connu d'utiliser des indicateurs de qualité d'image (ci-après appelés IQI) pour évaluer la qualité des images tomographiques acquises par l'appareil d'imagerie. Typiquement, ces IQI sont des fantômes (appelés aussi pièces étalons) comportant des inclusions et des aspérités jouant le rôle de défauts. En association avec un programme de calcul, ces fantômes aident ainsi à déterminer un ou plusieurs paramètres caractérisant la qualité des images tomographiques, tels que notamment la résolution spatiale selon différentes directions, le rapport signal/bruit, l'uniformité, etc.

Les fantômes connus à ce jour ne sont cependant pas adaptés au contrôle de la qualité d'images tomographiques de pièces, notamment celles en matériau composite. En particulier, ces fantômes présentent généralement une forme prismatique, ce qui entraîne des artéfacts dans les données tomographiques obtenues après la reconstruction 2D ou 3D de la pièce.

En outre, l'article HANSON K M ET AL: "Computed tomography using proton energy loss",PHYSICS IN MEDICINE AND BIOLOGY, INSTITUTE OF PHYSICS PUBLISHING, BRISTOL GB, vol. 26, no. 6, novembre 1981 (1981-11), pages 965-983, XP020022418,ISSN: 0031-9155, DOI: 10.1088/0031-9155/26/6/001 ; décrit un fantôme destiné à être utilisé pour le contrôle de la qualité d'images tomographiques.

Il existe donc un besoin de pouvoir disposer d'un fantôme pour le contrôle de la qualité en imagerie tomographique 2D ou 3D sur des pièces qui ne présente les inconvénients précités.

### Objet et résumé de l'invention

La présente invention répond notamment à ce besoin en proposant un fantôme destiné à être utilisé pour le contrôle de la qualité d'images tomographiques, comprenant :
une plaque cylindrique réalisée dans un matériau homogène ayant une densité d1, ladite plaque présentant un axe de révolution ;
deux cylindres insérés dans la plaque, les cylindres étant réalisés dans des matériaux homogènes ayant des densités d2, d3 différentes, la densité de l'un des cylindres étant supérieure à la densité d1 de la plaque et la densité de l'autre cylindre étant inférieure à la densité d1 de la plaque ; et
une première série de paires de trous de diamètres différents percés dans la plaque, les trous de la première série s'étendant suivant un axe parallèle à l'axe de révolution de la plaque, caractérisé en ce que le fantôme comprend en outre une deuxième série de paires de trous de diamètres différents percés dans la plaque, les trous de la deuxième série s'étendant suivant un axe perpendiculaire à l'axe de révolution de la plaque de manière à ce que lesdits trous de la deuxième série s'étendent radialement dans la plaque cylindrique.

Le fantôme selon l'invention est parfaitement adapté au contrôle de la qualité d'images tomographiques 2D ou 3D, notamment sur des pièces en matériau composite, sans perturber la qualité de l'image de la pièce contrôlée. La forme cylindrique de la plaque du fantôme permet de réduire les artéfacts tomographiques qui sont susceptibles d'être générés par le fantôme lui-même. De plus, le fantôme selon l'invention présente trois densités différentes, ce qui permet d'établir une courbe de calibration pour mesurer avec précision la densité de la pièce contrôlée.

Les trous percés dans la plaque du fantôme selon l'invention permettent de mesurer la résolution de la tomographie. La présence de trous présente l'avantage de pouvoir effectuer une telle mesure sans avoir à recourir à des fils métalliques de haute densité qui viendraient notamment rayonner par rapport au matériau de la pièce contrôlée.

Dans une application au contrôle de la qualité d'images tomographiques 3D, les trous d'une même paire de trous de la première série et de la deuxième série de paires de trous sont espacés l'un de l'autre d'une distance égale à leur diamètre

Dans cette application, les trous de la première série ont de préférence un diamètre identique aux trous de la deuxième série. De plus, les trous de la deuxième série peuvent déboucher sur un bord périphérique de la plaque à une même hauteur dudit bord périphérique. Dans ce cas, les trous de la deuxième série sont avantageusement répartis angulairement autour de l'axe de révolution de la plaque.

Quant aux trous de la première série, ils peuvent déboucher au niveau de deux faces opposées de la plaque, les paires de trous étant disposées selon des diamètres différents de la plaque. Dans ce cas, les paires de trous de la première série sont avantageusement disposées par ordre décroissant de diamètre entre la périphérie externe de la plaque et le centre de ladite plaque.

La plaque du fantôme peut être réalisée dans un matériau homogène ayant une densité d1 comprise entre 1,2 et 8 proche de la densité de la pièce contrôlée.

Les deux cylindres du fantôme insérés dans la plaque comprennent des axes de révolution respectifs qui peuvent être positionnés sur un même diamètre de la plaque.

L'invention a également pour objet une utilisation d'au moins un fantôme telle que défini précédemment pour le contrôle de la qualité en imagerie tomographique d'une aube de soufflante de moteur à turbine à gaz réalisée en matériau composite, dans laquelle la plaque présente un diamètre de 50mm environ et une épaisseur de 6mm environ, les deux cylindres ont chacun un diamètre d'environ 10mm et les trous de la première série de trous ont des diamètres compris entre 0,2mm et 1,2mm environ.

### Brève description des dessins

D'autres caractéristiques et avantages de la présente invention ressortiront de la description faite ci-dessous, en référence aux dessins annexés qui en illustrent un exemple de réalisation dépourvu de tout caractère limitatif. Sur les figures :
- les figures 1 et 2 sont des vues d'un fantôme selon l'invention, respectivement en perspective et de face ;
- la figure 3 est une loupe de la figure 2 montrant une série de trous percés dans la plaque du fantôme ; et
- la figure 4 est une vue de côté du fantôme des figures 1 et 2.

### Description détaillée de l'invention

L'invention s'applique au contrôle de la qualité d'images obtenues par imagerie tomographique 2D ou 3D, par exemple d'une pièce aéronautique en matériau composite telle qu'une aube de soufflante de moteur à turbine à gaz.

De façon connue en soi, la tomographie par absorption de rayons X est une technique non destructive qui permet la reconstruction d'images « en coupe » d'une pièce à trois dimensions. Son principe repose sur l'analyse multidirectionnelle de l'interaction d'un faisceau de rayons X avec la matière, par enregistrement avec des détecteurs du rayonnement transmis après traversée de la pièce à contrôler. Les données acquises lors de la prise de mesure sont collectées suivant des orientations multiples. À l'aide de ces données, une image numérique est calculée et reconstruite mathématiquement en valeurs d'amplitude dont chaque valeur traduit point par point le coefficient d'atténuation local du matériau. Celle-ci, après calibration et étalonnage, peut être traduite en échelle de densité.

Le contrôle de la qualité des images acquises par l'appareil d'imagerie tomographique est réalisé grâce à des fantômes (également appelés IQI pour « Indicateurs de Qualité d'Image ») qui sont placés ici systématiquement avec la pièce à contrôler lors de la prise de mesures. Ces fantômes ont pour but de mesurer la résolution des images acquises par tomographie en permettant de distinguer, sur ces images, deux éléments de petite taille éloignés l'un de l'autre. Les fantômes permettent également de mesurer la résolution de densité.

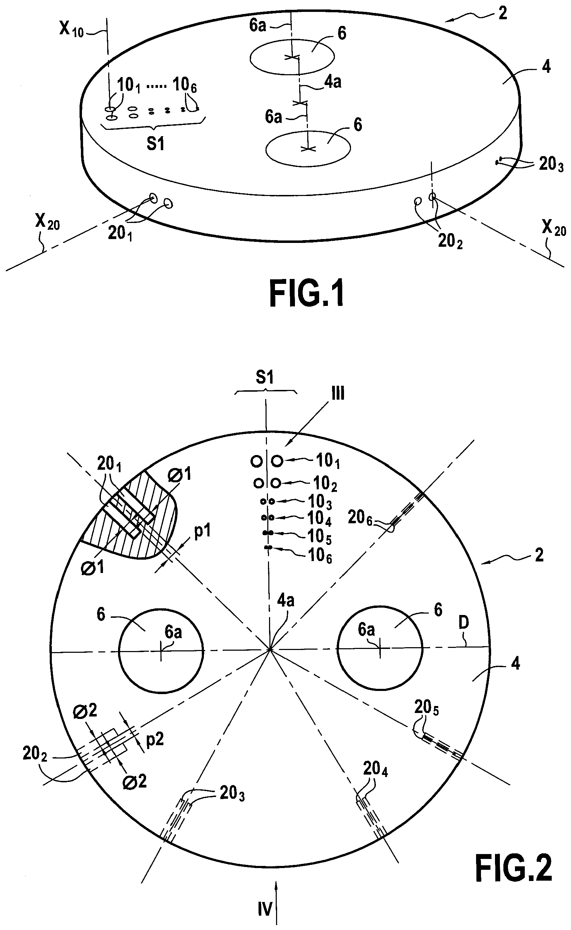

A cet effet, comme représenté sur les figures 1 à 3, le fantôme 2 selon l'invention comprend notamment une plaque cylindrique 4 dans laquelle sont insérés deux cylindres 6 et sont pratiqués deux séries S1, S2 de paires de trous.

De façon plus précise, la plaque 4 présente un axe de révolution 4a et est réalisée dans un matériau homogène, par exemple un matériau thermoplastique, ayant une densité d1 qui est voisine de la densité du matériau dans lequel est réalisée la pièce à contrôler.

A titre d'exemple, pour le contrôle d'une pièce en matériau composite ayant une densité d'environ 1,6 (ce qui peut être notamment le cas d'une aube de soufflante de turboréacteur réalisée en matériau composite), on choisira une densité d1 pour le matériau plastique de la plaque 2 qui est comprise entre 1,2 et 1,8.

Les deux cylindres 6 insérés dans la plaque présentent chacun un axe de révolution 6a. Ils sont également réalisés dans des matériaux plastiques homogènes ayant des densités respectives d2 et d3 qui sont différentes l'une de l'autre et qui encadrent la densité du matériau composite dans lequel est réalisée la pièce à contrôler (et donc la densité d1 de la plaque). En d'autres termes, les matériaux plastiques de la plaque 4 et des cylindres 6 insérés dans celle-ci sont choisis de telle sorte que : d2 (ou d3) < d1 < d3 (ou d2).

A titre d'exemple, pour un matériau composite ayant une densité d'environ 1,6, on choisira un matériau plastique ayant une densité d2 de 1,1 pour l'un des cylindres 6 et un matériau plastique ayant une densité d3 de 2,2 pour l'autre cylindre. A cet effet, les matériaux plastiques utilisés pour réaliser les cylindres pourront être du polytétrafluoroéthylène (PTFE) et un polyamide de type Nylon®.

Ainsi, le fantôme 2 selon l'invention est constitué de trois matériaux de densités différentes, ce qui permet d'établir une courbe de calibration pour mesurer la densité de la pièce contrôlée.

Comme représenté sur la figure 2, les axes de révolution 6a respectifs des deux cylindres 6 insérés dans la plaque sont parallèles à l'axe de révolution 4a de ladite plaque et sont avantageusement positionnés sur un même diamètre D de celle-ci.

Par ailleurs, comme indiqué précédemment, la plaque 4 du fantôme selon l'invention comprend également deux séries S1, S2 de paires de trous, à savoir une première série S1 de n paires de trous 10₁ à 10ₙ (six paires de trous 10₁ à 10₆ dans l'exemple illustré) ayant chacun un axe X₁₀ qui est orienté axialement (par rapport à l'axe de révolution 4a de la plaque), et une deuxième série S2 de n paires de trous 20₁ à 20ₙ (six paires de trous 20₁ à 20₆ dans l'exemple illustré) ayant chacun un axe X₂₀ qui est orienté radialement (par rapport à l'axe de révolution 4a de la plaque). Dans l'exemple décrit ici, l'axe X₁₀ (montré sur la figure 1 pour un des trous 10₁) correspond à l'axe suivant lequel les trous cylindrique 10₁ à 10₆ s'étendent, cet axe X₁₀ étant parallèle à l'axe de révolution 4a de la plaque 4 elle aussi cylindrique. Toujours dans l'exemple décrit ici, l'axe X₂₀ (montré sur la figure 1 pour un des trous 20₁ et un des trous 20₂) correspond à l'axe suivant lequel les trous cylindrique 20₁ à 20₆ s'étendent, cet axe X₂₀ étant perpendiculaire à l'axe de révolution 4a de la plaque 4 elle aussi cylindrique, l'axe X₂₀ pouvant par exemple être parallèle à un rayon de la plaque 4.

On notera que pour le contrôle de la qualité en imagerie tomographique 2D, seule la première série S1 de paires de trous est nécessaire, tandis que pour le contrôle de la qualité en imagerie tomographique 3D, les deux séries S1 et S2 de paires de trous est nécessaire.

Les trous d'une même paire (pour la première série S1 et la deuxième série S2) ont un même diamètre. En revanche, les paires de trous dans une même série ont des diamètres différents.

De plus, les trous 20₁ à 20ₙ de la deuxième série S2 sont espacés l'un de l'autre d'une distance égale à leur diamètre. Ainsi, sur l'exemple illustré par les figures, les deux trous 20₁ ont un même diamètre ∅1 et sont espacés l'un de l'autre d'une distance p1 égale à leur diamètre ∅1. De même, les trous 20₂ ont un même diamètre ∅2 et sont espacés d'une distance p2 qui est égale à leur diamètre ∅2, etc.

En outre, la profondeur des trous 20₁ à 20ₙ de cette deuxième série S2 ne doit pas être trop importante pour éviter de provoquer des artéfacts. A titre d'exemple, on choisira une profondeur d'environ 5mm.

De la même manière, les trous 10₁ à 10ₙ de la première série S1 sont espacés l'un de l'autre d'une distance (p1, p2, p3, ..., pn) égale à leur diamètre (∅1, ∅2, ∅3, ..., ∅n). Sur l'exemple illustré, on notera d'ailleurs que les trous 20₁ à 20₆ de la première série S1 ont un diamètre identique aux trous 20₁ à 20₆ de la deuxième série S2, c'est-à-dire que la paire de trous 10₁ de la première série S1 et la paire de trous 20₁ de la deuxième série S2 ont un même diamètre ∅1, etc.

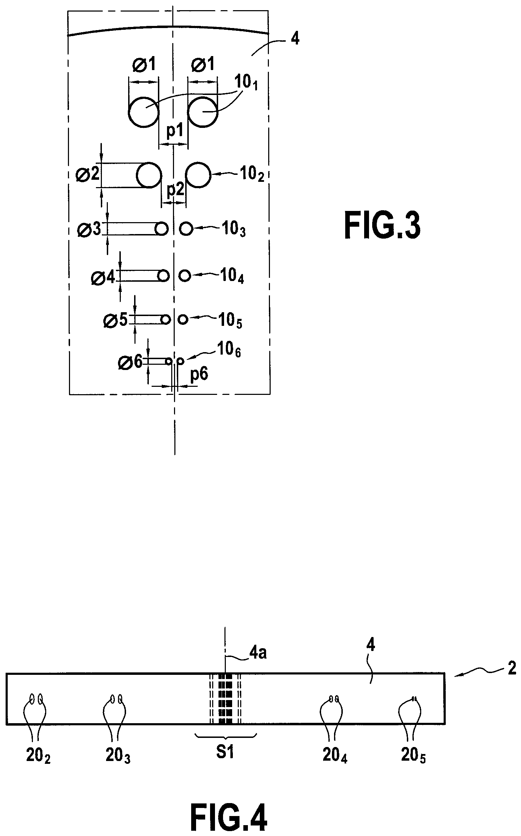

Comme représenté notamment sur les figures 2 et 4, les trous 20₁ à 20ₙ de la deuxième série S2 débouchent au niveau du bord périphérique de la plaque 4 à une même hauteur dudit bord périphérique (leurs axes respectifs sont alignés sur une même hauteur de la plaque prise dans le sens de son épaisseur). De plus, ces trous 20₁ à 20ₙ de la deuxième série S2 sont répartis angulairement autour de l'axe de révolution 4a de la plaque.

Quant aux trous 10₁ à 10ₙ de la première série S1, ils débouchent au niveau de deux faces opposées de la plaque 4 en la traversant de part en part dans le sens de son épaisseur (voir la figure 4).

De plus, les paires de trous 10₁ à 10ₙ de la première série S1 sont avantageusement disposées selon des diamètres différents de la plaque par ordre décroissant de diamètre depuis l'extérieur de la plaque vers l'intérieur de celle-ci. Ainsi, les trous 10₁ ayant le diamètre ∅1 le plus important sont disposés à l'extérieur de la plaque et les trous 10ₙ ayant le diamètre ∅n le plus petit sont disposés à l'intérieur de la plaque. Cette caractéristique permet ainsi d'augmenter la gamme de mesure du contraste.

Un exemple de dimensions pour la réalisation d'un fantôme utilisé dans le cadre du contrôle de la qualité d'images tomographiques d'une aube de soufflante de turboréacteur réalisé en matériau composite est donné dans le tableau ci-dessous (les valeurs sont en mm).

| | | | | | | | | |
|---|---|---|---|---|---|---|---|---|
| diamètre | épaisseur | diamètre | Ø1 | Ø2 | Ø3 | Ø4 | Ø5 | Ø6 |
| plaque | plaque | cylindres | p1 | p2 | p3 | p4 | p5 | p6 |
| 50 | 6 | 10 | 1,2 | 1,0 | 0,5 | 0,4 | 0,3 | 0,2 |

Des fantômes ayant de telles dimensions sont particulièrement avantageux pour réaliser un contrôle de la qualité d'images tomographiques d'une aube de soufflante de turboréacteur réalisé en matériau composite. A cet effet, deux fantômes ayant ces dimensions sont positionnés systématiquement avec l'aube à contrôler lors des mesures, l'un des fantômes étant placé sous le pied d'aube et l'autre fantôme étant placé en décalé au-dessus de la tête d'aube. La tomographie de l'aube est alors réalisée en quatre fois dans le sens de sa hauteur de sorte que la première et la quatrième images tomographiques contiennent l'un des fantômes pour exercer un contrôle de la qualité.

La résolution (et donc la qualité) des première et quatrième images tomographiques est contrôlée grâce à une mesure entre deux trous d'une même paire de trous (appartenant aux séries S1, S2) : si l'on constate une différence de valeurs d'amplitude supérieure à une valeur préétablie entre ces deux trous, il est considéré que l'image contrôlée présente un défaut de qualité. Par ailleurs, si l'on parvient à la conclusion que les première et quatrième images tomographiques ainsi contrôlées ne présentent pas de défaut, il peut être considéré que les deux images intermédiaires sont également exemptes de défaut.

## Revendications

1. Fantôme (2) destiné à être utilisé pour le contrôle de la qualité d'images tomographiques, comprenant :
une plaque cylindrique (4) réalisée dans un matériau homogène ayant une densité d1, ladite plaque cylindrique présentant un axe de révolution (4a) ;
deux cylindres (6) insérés dans la plaque, les cylindres étant réalisés dans des matériaux homogènes ayant des densités d2, d3 différentes, la densité de l'un des cylindres étant supérieure à la densité d1 de la plaque et la densité de l'autre cylindre étant inférieure à la densité d1 de la plaque ; et
une première série (S1) de paires de trous (10₁ à 10ₙ) de diamètres (∅1 à ∅n) différents percés dans la plaque, les trous de la première série s'étendant suivant un axe parallèle à l'axe de révolution de la plaque , **caractérisé en ce que** le fantôme comprend en outre une deuxième série (S2) de paires de trous (20₁ à 20ₙ) de diamètres (∅1 à ∅n) différents percés dans la plaque, les trous de la deuxième série s'étendant suivant un axe perpendiculaire à l'axe de révolution (4a) de la plaque de manière à ce que lesdits trous de la deuxième série s'étendent radialement dans la plaque cylindrique.

2. Fantôme selon la revendication 1, **caractérisé en ce que** les trous d'une même paire de trous de la première série (S1) et de la deuxième série (S2) de paires de trous sont espacés l'un de l'autre d'une distance (p1 à pn) égale à leur diamètre.

3. Fantôme selon la revendication 2, dans lequel les trous (10₁ à 10ₙ) de la première série (S1) ont un diamètre identique aux trous (20₁ à 20ₙ) de la deuxième série (S2).

4. Fantôme selon l'une quelconque des revendications 1 à 3, dans lequel les trous de la deuxième série (S2) débouchent sur un bord périphérique de la plaque à une même hauteur dudit bord périphérique.

5. Fantôme selon la revendication 4, dans lequel les trous de la deuxième série (S2) sont répartis angulairement autour de l'axe de révolution de la plaque.

6. Fantôme selon l'une quelconque des revendications 1 à 5, dans lequel les trous de la première série (S1) débouchent au niveau de deux faces opposées de la plaque, les paires de trous étant disposées selon des diamètres différents de la plaque.

7. Fantôme selon la revendication 6, dans lequel les paires de trous de la première série (S1) sont disposées par ordre décroissant de diamètre entre la périphérie externe de la plaque et le centre de ladite plaque.

8. Fantôme selon l'une quelconque des revendications 1 à 7, dans lequel la plaque est réalisée dans un matériau homogène ayant une densité d1 comprise entre 1,2 et 8.

9. Fantôme selon l'une quelconque des revendications 1 à 8, dans lequel les deux cylindres (6) insérés dans la plaque (4) comprennent des axes de révolution respectifs (6a) qui sont positionnés sur un même diamètre (D) de la plaque.

10. Utilisation d'au moins un fantôme (2) selon l'une quelconque des revendications 1 à 9 pour le contrôle de la qualité en imagerie tomographique d'une aube de soufflante de moteur à turbine à gaz réalisée en matériau composite, dans laquelle la plaque présente un diamètre de 50mm environ et une épaisseur de 6mm environ, les deux cylindres ont chacun un diamètre d'environ 10mm et les trous de la première série de trous ont des diamètres compris entre 0,2mm et 1,2mm environ.

## Patentansprüche

1. Phantom (2) zur Verwendung für die Qualitätskontrolle von Tomographiebildern, umfassend:
eine zylindrische Platte (4), die aus einem homogenen Material mit einer Dichte d1 ausgebildet ist, wobei die zylindrische Platte eine Rotationsachse (4a) aufweist,
zwei Zylinder (6), die in die Platte eingesetzt sind, wobei die Zylinder aus homogenen Materialien mit unterschiedlichen Dichten d2, d3 bestehen, wobei die Dichte von einem der Zylinder größer ist als die Dichte d1 der Platte, und wobei die Dichte des anderen Zylinders kleiner ist als die Dichte d1 der Platte, und
eine erste Reihe (S1) von Paaren von Löchern (10₁ bis 10ₙ) mit unterschiedlichen Durchmessern (∅1 bis ∅n), die in die Platte gebohrt sind, wobei sich die Löcher der ersten Reihe entlang einer zu der Rotationsachse der Platte parallelen Achse erstrecken, **dadurch gekennzeichnet, dass** das Phantom ferner eine zweite Reihe (S2) von Paaren von Löchern (20₁ bis 20ₙ) mit unterschiedlichen Durchmessern (∅1 à ∅n), welche in die Platte gebohrt sind, umfasst, wobei sich die Löcher der zweiten Reihe entlang einer zu der Rotationsachse (4a) der Platte senkrechten Achse erstrecken, so dass sich die Löcher der zweiten Reihe in der zylindrischen Platte radial erstrecken.

2. Phantom nach Anspruch 1, **dadurch gekennzeichnet, dass** die Löcher eines gleichen Paares von Löchern der ersten Reihe (S1) und der zweiten Reihe (S2) von Paaren von Löchern um einen Abstand (p1 bis pn), der gleich ihrem Durchmesser ist, voneinander beabstandet sind.

3. Phantom nach Anspruch 2, bei dem die Löcher (10₁ bis 10ₙ) der ersten Reihe (S1) einen Durchmesser aufweisen, der mit den Löchern (20₁ bis 20ₙ) der zweiten Reihe (S2) identisch ist.

4. Phantom nach einem der Ansprüche 1 bis 3, bei dem die Löcher der zweiten Reihe (S2) an einem Umfangsrand der Platte auf einer gleichen Höhe des Umfangsrandes ausmünden.

5. Phantom nach Anspruch 4, bei dem die Löcher der zweiten Reihe (S2) um die Rotationsachse der Platte winkelmäßig verteilt sind.

6. Phantom nach einem der Ansprüche 1 bis 5, bei dem die Löcher der ersten Reihe (S1) im Bereich von zwei gegenüberliegenden Seiten der Platte ausmünden, wobei die Löcherpaare in unterschiedlichen Durchmessern der Platte angeordnet sind.

7. Phantom nach Anspruch 6, bei dem die Löcherpaare der ersten Reihe (S1) in absteigender Durchmesser-Reihenfolge zwischen dem Außenumfang der Platte und dem Mittelpunkt der Platte angeordnet sind.

8. Phantom nach einem der Ansprüche 1 bis 7, bei dem die Platte aus einem homogenen Material mit einer Dichte d1 im Bereich zwischen 1,2 und 8 ausgebildet ist.

9. Phantom nach einem der Ansprüche 1 bis 8, bei dem die zwei Zylinder (6), die in die Platte (4) eingesetzt sind, jeweilige Rotationsachsen (6a) aufweisen, die auf einem gleichen Durchmesser (D) der Platte positioniert sind.

10. Verwendung von wenigstens einem Phantom (2) nach einem der Ansprüche 1 bis 9 zur Qualitätskontrolle bei der tomographischen Bildgebung einer aus Verbundwerkstoff gefertigten Gebläseschaufel eines Gasturbinentriebwerks, bei der die Platte einen Durchmesser von etwa 50 mm und eine Dicke von etwa 6 mm aufweist, die zwei Zylinder jeweils einen Durchmesser von etwa 10 mm aufweisen und die Löcher der ersten Reihe von Löchern Durchmesser im Bereich zwischen etwa 0,2 mm und etwa 1,2 mm aufweisen.

## Claims

1. A phantom (2) for use in quality control of tomographic images, and comprising:
• a cylindrical plate (4) made of a uniform material having density d1, said cylindrical plate presenting an axis of revolution (4a);
• two cylinders (6) inserted in the plate, the cylinders being made of uniform material having different densities d2, d3, the density of one of the cylinders being greater than the density d1 of the plate, and the density of the other cylinder being less than the density d1 of the plate; and
• a first series (S1) of pairs of holes (10₁ to 10ₙ) of different diameters (∅1 to ∅n) drilled in the plate, the holes of the first series extending along axes that are parallel to the axis of revolution of the plate, the phantom being **characterized in that** it further comprises a second series (S2) of pairs of holes (20₁ to 20ₙ) of different diameters (∅1 to ∅n) drilled in the plate, the holes of the second series extending along axes perpendicular to the axis of revolution (4a) of the plate so that said holes of the second series extend radially in the cylindrical plate.

2. A phantom according to claim 1, **characterized in that** the holes in any given pair of holes of the first series (S1) or of the second series (S2) of pairs of holes are spaced apart from each other by a distance (p1 to pn) equal to their diameter.

3. A phantom according to claim 2, wherein the holes (10₁ to 10ₙ) of the first series (S1) are of diameters that are identical to the holes (20₁ to 20ₙ) of the second series (S2).

4. A phantom according to any one of claims 1 to 3, wherein the holes of the second series (S2) open out into a peripheral edge of the plate at the same height of said peripheral edge.

5. A phantom according to claim 4, wherein the holes of the second series (S2) are angularly distributed around the axis of revolution of the plate.

6. A phantom according to any one of claims 1 to 5, wherein the holes of the first series (S1) open out into two opposite faces of the plate, the pairs of holes being arranged on different diameters of the plate.

7. A phantom according to claim 6, wherein the pairs of holes of the first series (S1) are arranged in order of decreasing diameter between the outer periphery of the plate and the center of said plate.

8. A phantom according to any one of claims 1 to 7, wherein the plate is made of a uniform material having a relative density d1 lying in the range 1.2 to 8.

9. A phantom according to any one of claims 1 to 8, wherein the two cylinders (6) inserted in the plate (4) have respective axes of revolution (6a) that are positioned on a common diameter (D) of the plate.

10. The use of at least one phantom (2) according to any one of claims 1 to 9, for quality control in tomographic imaging of a gas turbine engine fan blade made out of composite material, wherein the plate presents a diameter of about 50 mm and a thickness of about 6 mm, each of the two cylinders has a diameter of about 10 mm, and the holes of the first series of holes have diameters lying in the range 0.2 mm to 1.2 mm, approximately.
